**Europäisches Patentamt**

**European Patent Office**

**Office européen des brevets**

(19)

(11) Veröffentlichungsnummer : **0 357 969 B1**

(12)

# EUROPÄISCHE PATENTSCHRIFT

(45) Veröffentlichungstag der Patentschrift :
**30.09.92 Patentblatt 92/40**

(51) Int. Cl.$^5$ : **C07H 15/04**

(21) Anmeldenummer : **89114438.8**

(22) Anmeldetag : **04.08.89**

(54) **Verfahren zur Herstellung von Alkylglucosidverbindungen aus Oligo-und/oder Polysacchariden.**

Verbunden mit 89908994.0/0428579 (europäische Anmeldenummer/Veröffentlichungsnummer) durch Entscheidung vom 03.12.91.

(30) Priorität : **13.08.88 DE 3827534**

(43) Veröffentlichungstag der Anmeldung :
**14.03.90 Patentblatt 90/11**

(45) Bekanntmachung des Hinweises auf die Patenterteilung :
**30.09.92 Patentblatt 92/40**

(84) Benannte Vertragsstaaten :
**AT BE CH DE ES FR GB IT LI NL**

(56) Entgegenhaltungen :
**EP-A- 0 035 589**
**EP-A- 0 099 183**
**FR-A- 2 017 240**
**US-A- 2 390 507**
**US-A- 3 219 656**

(73) Patentinhaber : **Henkel Kommanditgesellschaft auf Aktien Henkelstrasse 67 W-4000 Düsseldorf 13 (DE)**

(72) Erfinder : **Wüst, Willi, Dr. Fasanering 32 W-4030 Ratingen (DE)**
Erfinder : **Eskuchen, Rainer, Dr. Benrather Schlossalle 36 W-4000 Düsseldorf-Benrath (DE)**
Erfinder : **Wollmann, Josef Kloster Strasse 112 W-5120 Herzogenrath j3 (DE)**
Erfinder : **Hill, Karlheinz, Dr. Am Hasenbusch 1 W-4006 Erkrath (DE)**
Erfinder : **Biermann, Manfred, Dr. Markscheiderhof 25 W-4330 Mülheim (DE)**

EP 0 357 969 B1

## Beschreibung

Die Herstellung von oberflächenaktive Alkylglucosidverbindungen enthaltenden Reaktionsprodukten ist in zahlreichen Druckschriften beschrieben. Alkylglucoside sind bekanntlich Acetale aus Zuckern und monofunktionellen Alkoholen. Als Tensidkomponenten sind insbesondere bedeutungsvoll überwiegend Monoglucoside enthaltende Reaktionsprodukte aus monofunktionellen aliphatischen Alkoholen mit 8 bis 22, insbesondere 12 bis 18 Kohlenstoffatomen und Glucose als Monosaccharid. Die entsprechenden Reaktionsgemische enthalten je nach Herstellung untergeordnete Mengen an entsprechenden Acetalen, die den Rest des längerkettigen monofunktionellen Alkohols an Oligosaccharidreste gebunden enthalten. Der Begriff Alkylglucosid soll hier sowohl die Alkylmonoglucoside als auch Alkyloligo- bzw. Alkylpolyglucoside, sowie entsprechende Alkenyl-Verbindungen umfassen, sofern es nicht ausdrücklich auf die jeweiligen strukturellen Unterschiede ankommt.

Die oberflächenaktiven Alkylglucoside mit $C_8$-$C_{22}$-, insbesondere mit $C_{12}$-$C_{18}$-Alkyl- bzw. Alkenylresten gehören zum Typ der nichtionischen Tenside. Ihr auf die längerkettigen monofunktionellen Alkohole zurückgehender Molekülbestandteil führt auf entsprechende Alkohole natürlichen und/oder synthetischen Ursprungs zurück. Das für die Praxis wichtigste Saccharid-Einsatzmaterial sind in der Natur vielfältig zur Verfügung stehenden Polyanhydroglucoseverbindungen, deren einzelne Glucoseeinheiten in Alpha-glucosidischer Verknüpfung vorliegen. Das wichtigste Einsatzmaterial natürlichen Ursprungs dieser Art ist die Stärke, die weltweit durch Nutzpflanzen unterschiedlichster Art, wie beispielsweise Kartoffeln, Mais, Tapioka, Reis, Weizen und dergleichen gebildet wird. Pulverförmige Stärken und ihre partiellen Abbauprodukte beispielsweise in Form eines entsprechenden, meist hochkonzentrierten Glucosesirups, stehen als vergleichsweise preiswerte Einsatzmaterialien zur Verfügung.

Im Rahmen der zahlreichen Versuche, wasch- bzw. reinigungsaktive Alkylglucosidverbindungen daraus herzustellen, sind die Probleme dieser Stoffklasse der Fachwelt bewußt geworden. Es gelingt nur mit Schwierigkeiten, Alkylmonoglucoside dieser Art in der von der Praxis geforderten Reinheit, d. h. mit hellen Farbwerten und hoher Farbbeständigkeit im Alkalischen zu gewinnen. Die hohe Empfindlichkeit des Polyanhydroglucose-Einsatzmaterials gegenüber den beim Verfahren benötigten hohen Temperaturen und Drucken unter Einfluß von Katalysatoren hat bis heute kein Verfahren bekannt werden lassen, bei dem im technischen Maßstab in einem Verfahrenszug Alkylglucosid enthaltende Reaktionsprodukte beispielsweise aus pulverförmiger Stärke hergestellt werden können.

Die einschlägige Literatur zur Herstellung der gewünschten waschaktiven Alkylglucoside befaßt sich insbesondere mit zwei Hilfselementen, die im Rahmen der Synthese in verschiedenartigster Weise variiert eingesetzt werden. Zunächst einmal wird vorgeschlagen, nicht die Polyanhydroglucosekomponenten als solche einzusetzen, sondern erst dieses Einsatzmaterial natürlichen Ursprungs zum Monosaccharid, d. h. zur Glucose abzubauen. Die Glucose kann dann als wasserfreies Material oder als Glucosehydrat der Acetalisierung zugeführt werden.

Das zweite von der Praxis eingesetzte Hilfsmittel benutzt die Tatsache, daß niedere monofunktionelle Alkohole, insbesondere solche des C-Zahlbereichs bis $C_6$ und insbesondere $C_3$-$C_5$ vergleichsweise einfach zur gewünschten Acetalisierung am Monosaccharid führen. Die so gebildeten Acetale des Monosaccharids besitzen allerdings nur unzureichende Tensideigenschaften. Sie werden durch Umacetalisierung mit den längerkettigen Alkoholen des Bereichs von $C_{12-18}$ in die gewünschten Alkylglucosid-Reaktionsprodukte mit oberflächenaktiven Eigenschaften umgewandelt. Auch dieser scheinbar vergleichsweise einfache Herstellungsweg ist für die Praxis mit zahlreichen einschränkenden Schwierigkeiten der eingangs genannten Art verbunden. Die Gewinnung hellfarbiger, farbstabiler und insbesondere im Alkalischen farbstabiler Alkylglucoside fordert bis heute einen so beträchtlichen Arbeitsaufwand, daß schon allein aus Kostengesichtspunkten diese Klasse wasch- und reinigungsaktiver Tensidkomponenten keine nennenswerte praktische Anwendung gefunden hat.

Aus dem umfangreichen Stand der Technik seien nur wenige Beispiele zitiert: Die US-Patentschrift 3,219,656 beschreibt die Herstellung von langkettigen Alkylglucosidgemischen mittels einer Reihe aufeinanderfolgender Alkohol-Austauschreaktionen. Das Verfahren geht von Glucose aus. Die US-Patentschrift 3,547,828 beschreibt die Herstellung eines ternären Gemisches aus Alkyloligoglucosiden, Alkylmonoglucosiden und den entsprechenden $C_{11}$-$C_{32}$-Alkanolen nach dem Umacetalisierungsverfahren mit Butanol. Auch hier wird zunächst Glucose sauer katalysiert mit Butanol zu Alkylglucosid umgesetzt, das anschließend einer Umacetalisierung zugeführt wird. Die US-Patentschrift 3,598,865 beschreibt die Umwandlung von Glucose oder Methylglucosid zu langkettigen Glucosidverbindungen in Gegenwart von primären und sekundären Alkoholen mit 3 bis 5 C-Atomen.

In der US-Patentschrift 4,223,129 wird die Umwandlung von Stärke natürlichen Feuchtigkeitsgehalts zu Methylglucosid ohne Vortrocknung der Stärke beschrieben. Die europäische Patentanmeldung 0 099 183 beschreibt die Umwandlung von Sacchariden, insbesondere Polysacchariden wie Stärke, mit Alkoholen mit wenigstens 3 C-Atomen zu Glucosidgemischen. Hierbei soll die Umsetzung in Gegenwart von wenigstens 2 Mol Wasser je molare Saccharideinheit in Gegenwart von insbesondere sauren Katalysatoren durchgeführt werden. Bevorzugt enthält die zur Dispergierung der Polysaccharidverbindung eingesetzte Flüssigphase zusätzlich ein alkohol-lösliches organisches Hilfslösungsmittel. Als besonders geeignete Alkohole zur Bildung der Alkylglucosidverbindungen sind die Alkanole mit 3 bis 6 C-Atomen und insbesondere mit 3 oder 4 C-Atomen herausgestellt, wobei dem Butanol besondere Bedeutung gegeben wird. Die auf diese Weise gebildeten niederen Alkylglucoside können nach den Angaben dieser Druckschrift als Zwischenverbindungen bei der Herstellung von oberflächenaktiven Alkylglucosidverbindungen eingesetzt werden, ohne daß jedoch nähere Einzelheiten zu diesem nachfolgenden Reaktionsschritt angegeben sind.

In der deutschen Patentanmeldung P 37 23 826.4 wird ein Mehrstufenverfahren zur Herstellung von oberflächenaktiven Alkylglucosiden beschrieben, bei dem Glucose zunächst mit Butanol zu einem intermediärem Reaktionsprodukt umgesetzt und dieses dann mit höheren Alkoholen des überwiegenden C-Zahlbereichs von $C_{12-18}$ umacetalisiert wird.

Die Erfindung geht von der Aufgabe aus, die als natürliche Einsatzmaterialien wohlfeil zur Verfügung stehenden Polyglucosidverbindungen von der Art der Stärken oder ihrer partiellen Abbauprodukte in einem Verfahrenszuge in die angestrebten Alkylglucosidverbindungen mit oberflächenaktiven Eigenschaften umzuwandeln, ohne daß es der Isolierung irgendwelcher Zwischenstufen bedarf. Zwar arbeitet auch das erfindungsgemäße Verfahren in sich mehrstufig, es ist aber so ausgelegt, daß es - bevorzugt unter wenigstens anteilsweiser Kreislaufführung zwischenzeitlich benötigter Reaktanten - als eine in sich geschlossene Verfahrenseinheit vom Polysaccharid unmittelbar zum wasch- bzw. reinigungsaktiven Alkylglucosid führt. Dabei fallen alkylglucosidische Reaktionsprodukte an, die dem geforderten Standard an Hellfarbigkeit, Farbstabilität und Alkalistabilität genügen. Der mit der erfindungsgemäßen Herstellung verbundene Kostenaufwand wird durch die erfindungsgemäßen Maßnahmen so eingeschränkt, daß jetzt das Preisniveau von alternativ in der Praxis eingesetzten Tensidkomponenten erreicht wird.

Gegenstand der Erfindung ist dementsprechend ein Verfahren zur Herstellung von oberflächenaktive Alkylglucosidverbindungen enthaltenden Reaktionsprodukten mit überwiegendem Gehalt an Alkyl-Monoglucosid aus Oligo- und/oder Polysacchariden, insbesondere Stärke oder partiellen Stärkeabbauprodukten, und längerkettigen monofunktionellen Alkoholen unter Einschluß einer intermediären Bildung von Niedrig-Alkyl-Glucosiden und deren Umacetalisierung mit den längerkettigen monofunktionellen Alkoholen. Das erfindungsgemäße Verfahren ist dadurch gekennzeichnet, daß man

a) das Saccharid-Einsatzmaterial mit solchen Mengen einer bei Verfahrenstemperatur 1-phasigen Wasser/ Niedrig-Alkanol-Mischung bei Temperaturen oberhalb 100 °C und erhöhten Drucken sauer katalysiert umsetzt, daß während des ganzen Reaktionsablaufs die Rühr- und Fließfähigkeit des Reaktionsgemisches erhalten bleibt und dabei die Umsetzung so weit führt, daß ein flüssiges, praktisch klares 1-phasiges erstes Reaktionsprodukt anfällt, woraufhin man

b) dieses erste Reaktionsprodukt, gegebenenfalls nach Vorbehandlung zur Farbaufhellung mit einem Adsorptionsmittel, z. B. Aktivkohle oder Ionenaustauscher, mit den auf erhöhte Temperaturen vorgeheizten längerkettigen Alkoholen ebenfalls unter Ausbildung eines während des gesamten Reaktionsablaufs bei Verfahrenstemperatur im Flüssiganteil 1-phasigen zweiten Reaktionsgemisches unter solchen Reaktionsbedingungen von Druck, Temperatur und Zugabegeschwindigkeit zusammenführt, daß der jeweils eingebrachte Wasseranteil praktisch unmittelbar in die Dampfphase übergeht und dort zusammen mit begleitendem Niedrig-Alkanol kondensiert und damit dem Reaktionsgeschehen entzogen wird, woraufhin

c) unter Aufrechterhaltung der 1-Phasigkeit des Flüssiganteils darin noch vorliegendes und/oder durch Umacetalisierung freigesetztes Niedrig-Alkanol abgedampft und das so gebildete zweite Reaktionsprodukt gewünschtenfalls in an sich bekannter Weise - insbesondere durch Neutralisation, Entfernung überschüssigen längerkettigen Alkohols und/oder Bleiche weiterführend gereinigt wird.

Das erfindungsgemäße Verfahren verwirklicht damit die gestellte Aufgabe mittels einer Kombination von Verfahrensstufen, die in optimaler Weise einerseits den jeweils angestrebten Umsatz ermöglichen, andererseits ein Reaktionsgemisch liefern, das in der nachfolgenden Verfahrensstufe als Einsatzmaterial - bei geeigneter Handhabung im Sinne der erfindungsgemäßen Lehre - störungsfrei in Richtung auf das angestrebte Endprodukt weiterverarbeitet werden kann. Die Besonderheiten und bevorzugten Elemente des erfindungsgemäßen Handelns werden im nachfolgenden in der Sequenz des Mehrstufenverfahrens beschrieben.

Als Glucoseeinsatzmaterial sind Polysaccharidverbindungen insbesondere natürlichen Ursprungs geeignet, die Alpha-glucosidisch verknüpfte Polyanhydroglucoseeinheiten, insbesondere in mehr oder weniger langer Kette enthalten. Das bevorzugte Polysaccharid für die Verarbeitung im erfindungsgemäßen Verfahren ist Stärke beliebigen Ursprungs, insbesondere native Stärke aus Kartoffeln, Mais, Tapioka, Reis, Weizen und dergleichen und/oder ihre partiellen Abbauprodukte, wie sie in Form meist hochkonzentrierter wäßriger sirupartiger Produkte als Polyglucose- oder Glucose-Sirup wohlfeil zur Verfügung stehen. Irgendeiner besonderen Vorbehandlung, insbesondere einer Trocknung des Einsatzmaterials bedarf es nicht, da ja im Gegenteil als wesentliches Element das erfindungsgemäße Verfahren in der ersten Stufe die Mitverwendung beträchtlicher Wassermengen vorsieht. Auch die im Handel befindlichen wasserhaltigen sirupartigen Ausgangsmaterialien bedürfen im allgemeinen noch des Zusatzes von Wasser, um für die erste Stufe des erfindungsgemäßen Verfahrens geeignet zu sein.

In dieser ersten Verfahrensstufe wird das Saccharid-Einsatzmaterial mit solchen Mengen einer bei Verfahrenstemperatur 1-phasigen Wasser/Niedrig-Alkanol-Mischung bei Temperaturen oberhalb 100 °C und erhöhten Drucken in Gegenwart eines sauren Katalysators umgesetzt, daß die Rühr- und Fließfähigkeit des Reaktionsgemisches während des ganzen Reaktionsablaufs erhalten bleibt. Hier sind insbesondere die folgenden Gesichtspunkte zu berücksichtigen:

Das Stärkepartikel unterliegt bei Einsatz von Wasser und den erfindungsgemäßen Bedingungen Einwirkungen, die einerseits gewünscht sind, andererseits aber das Verfahren gefährden. Primärer und gewünschter Effekt ist der Aufbruch der quasi-kristallinen Tertiärstruktur natürlicher Stärkepartikel durch Anquellen und Verkleistern der Stärkemoleküle unter dem Wassereinfluß. Die damit verbundene Gefährdung des Verfahrens liegt in der Verkleisterung der Stärke und damit in einer partiellen oder vollständigen Erstarrung des Reaktionsgemisches im ersten Verfahrensschritt. Erfindungsgemäß wird dieser Gefahr dadurch entgegengewirkt, daß so hinreichende Mengen der wäßrig alkoholischen Flüssigphase eingesetzt werden, daß bei Verarbeitungstemperaturen während des ganzen Reaktionsablaufs die Rühr- und Fließfähigkeit des Reaktionsgemisches sichergestellt ist. Zahlenmäßig festgelegte Einzelheiten zu diesem Verfahrensschritt werden weiter unten noch angegeben. Die Funktionsfähigkeit dieses ersten Verfahrensschrittes wird weiterhin dadurch gefördert, daß das Wasser in Form einer bei Verfahrenstemperatur 1-phasigen Wasser/Niedrig- Alkanol-Mischung eingesetzt wird. Der Wasseranteil dieser Flüssigphase bricht zunächst die Kornstruktur des Stärkepartikels auf und führt vermutlich wenigstens zur partiellen Hydrolyse des Polysaccharids in Richtung auf Monosaccharid. Die in homogener Mischung gleichzeitig vorliegende Niedrig-Alkanol-Komponente führt unter den Verfahrensbedingungen zur wenigstens anteilsweisen Acetalisierung der gebildeten Glucose bzw. Oligo-Glucosekomponenten. Vermutlich wird auf diese Weise die Löslichkeit der entstehenden Reaktionsgemische in der eingesetzten Flüssigphase und damit die Beweglichkeit dieses ersten Reaktionsansatzes verbessert.

In der bevorzugten Ausführungsform der Erfindung wird wenigstens während substantieller Zeitabschnitte dieser ersten Verfahrensstufe die intensive Vermischung des flüssigen Reaktionsgutes vorgesehen, wozu bei Laboransätzen übliche Rühreinrichtungen für Autoklaven (ca. 200 U/min) ausreichen. Bei Ansätzen im großen Maßstab werden als hochwirksame Rührvorrichtungen Inline-Mischelemente, wie zum Beispiel Supratonmischer, bevorzugt eingesetzt.

Die Umsetzung zwischen eingesetztem Poly- und/oder Oligosaccharid, Wasser und Niedrig-Alkanol erfolgt unter Einwirkung von saurer Katalyse. Sowohl hier wie in den nachfolgenden Verfahrensstufen sind die im Zusammenhang mit der Herstellung von Alkylglucosiden zahlreich beschriebenen sauren Katalysatoren auf Basis anorganischer und/oder organischer Säuren geeignet. Besonders geeignete saure Katalysatoren sind Sulfonsäuren, insbesondere aromatische Sulfonsäuren. Ein herausragender Vertreter ist hier die p-Toluolsulfonsäure.

Für die Herstellung hinreichend hellfarbiger Reaktionsendprodukte ist es bedeutungsvoll, von Anfang an im Mehrstufenverfahren Reaktionsbedingungen zu wählen, die eine vergleichsweise milde Einwirkung überall dort erlauben, wo bei intensiver Einwirkung unerwünschte Dunkel-Verfärbungen zu erwarten sind. Bedeutungsvoll ist in diesem Zusammenhang insbesondere die Aufheizung des Reaktionsgemisches und die Erhaltung des gewünschten Temperaturniveaus. Es kann im Rahmen der Erfindung bevorzugt sein, hier vergleichsweise milde Verfahrensbedingungen - trotz der an sich geforderten Verfahrenstemperatur oberhalb 100 °C - dadurch zu schaffen, daß die Aufheizung des Reaktionsgemisches an einem im Kreislauf geführten Teilstrom außerhalb des Hauptreaktors erfolgt. Durch Einsatz vergleichsweise großer Wärmeaustauschflächen in diesem Teilstrom kann im eingesetzten Heizmedium mit Temperaturen gearbeitet werden, die nur beschränkt oberhalb der geforderten Verfahrenstemperatur liegen und gleichwohl eine hinreichend rasche Aufheizung des Reaktionsgemisches sichergestellt sein. Einzelheiten zu dieser bevorzugten Maßnahme im Sinne des erfindungsgemäßen Handelns finden sich bei der nachfolgenden Erörterung der zweiten Verfahrensstufe.

Die in homogener Mischung mit Wasser in dieser ersten Verfahrensstufe eingesetzten monofunktionellen niederen Alkanole sollen in der bevorzugten Ausgestaltung der Erfindung mit Wasser Azeotrope ausbilden, die in der nachfolgenden Reaktion die Abtrennung eines Wasser/Alkoholgemisches aus dem Reaktionsgeschehen erleichtern. Geeignet sind insbesondere die niederen Alkohole mit 3 bis 6, insbesondere 3 bis 5 C-Atomen. Besondere Bedeutung kommt hier wiederum den Vertretern zu, die heterogene Azeotrope bilden können, d. h. deren Azeotrop bei der Kondensation zur spontanen Phasentrennung führt. Auf diese Weise kann in nachfolgenden Reaktionsschritten das in das Verfahren eingetragene Wasser ausgekreist werden, während gleichzeitig wenigstens der überwiegende Anteil des monofunktionellen Alkohols in den Verfahrenskreislauf zurückgeführt werden kann. Die Butanole sind die wichtigsten Alkohole im Sinne der Erfindung, besondere Bedeutung hat das n-Butanol.

Unter den erfindungsgemäß in der ersten Verfahrensstufe eingesetzten Verfahrensbedingungen von Temperatur und Druck gelingt innerhalb vergleichsweise kurzer Zeit von etwa 15 bis 30 Minuten die Ausbildung eines Reaktionsgemisches, das sich als flüssiges, praktisch klares 1-phasiges erstes Reaktionsprodukt darstellt. Die Ausbildung eines solchen 1-phasigen Reaktionsproduktes und die Beibehaltung der 1-Phasigkeit des eingesetzten Flüssigmaterials während des Gesamtverfahrens ist für die Herstellung von hochwertigen Alkylglucosidprodukten der gewünschten Art von wichtiger Bedeutung. Das Auftreten von Phasentrennungen führt in der Regel zu einer Entgleisung des Reaktionsablaufs und zur Bildung von unerwünschten Nebenprodukten, die in nachfolgenden Verfahrensschritten nicht mehr zum gewünschten Alkylglucosid aufbereitet werden können. Insbesondere wird auch die Farbgebung des Reaktionsproduktes negativ beeinflußt. Weitere Schwierigkeiten können in der unerwünschten Belegung der Reaktorinnenwände mit zähflüssigen Polyglucosesirupen liegen. Die erfindungsgemäß geforderte 1-Phasigkeit im eingesetzten Flüssiganteil und letztlich die Bildung eines praktisch klaren 1-phasigen ersten Reaktionsproduktes verhindern Störungen dieser Art, wobei sich diese 1-Phasigkeit auf das erste Reaktionsprodukt unter Reaktionsbedingungen bezieht.

Der Saccharidaufschluß der ersten Reaktionsstufe wird vorzugsweise bei Temperaturen oberhalb 125 °C vorgenommen, wobei das Arbeiten im Bereich von etwa 135 bis 170 °C, vorzugsweise 140 bis 160, und insbesondere im Bereich von etwa 140 bis 150 °C bevorzugt ist. Zweckmäßigerweise wird bei diesem Aufschluß unter wenigstens anteilsweiser Bildung der Niedrig-Alkylglucoside bei dem sich im geschlossenen System ausbildenden Eigendruck gearbeitet. Im bevorzugten Temperaturbereich liegt dieser Eigendruck etwa im Bereich von 4 bis 10 bar, insbesondere 4 bis 8 und ganz besonders im Bereich von etwa 4,5 bis 7 bar. Unter diesen Verfahrensbedingungen gelingt es beispielsweise, native Stärkepulver in der vergleichsweise kurzen Reaktionszeit von etwa 15 bis 30 Minuten bei Verfahrenstemperatur zum erfindungsgemäß geforderten 1-phasigen Reaktionsprodukt aufzuschließen, das in die nachfolgenden Verfahrensschritte eingespeist werden kann.

Zum Ansetzen des ersten Reaktionsgemisches wird es bevorzugt, Wasser und den monofunktionellen niedrigen Alkohol - insbesondere n-Butanol - in etwa gleichen Molmengen zum Einsatz zu bringen. Es ist weiterhin bevorzugt, im ersten Reaktionsgemisch Molverhältnisse von eingesetzter Saccharidverbindung : Wasser im Bereich von etwa 1 : 5 bis 12, vorzugsweise 1 : 6 bis 12, insbesondere im Bereich von etwa 1 : 6 bis 9, und ganz besonders von etwa 1 : 8 zu verwenden. Diese Mengenberechnungen beziehen sich dabei auf die jeweils eingesetzte Saccharidverbindung (berechnet als Anhydroglucose, Molekulargewicht 162) im wasserfreien Zustand, d. h. die Wassermenge ist bei der Berechnung mit zu berücksichtigen, die von dem Saccharideinsatzmaterial in die Reaktion mit eingebracht wird, sei es in Form des natürlichen Wassergehalts von Stärken (üblicherweise etwa 12 bis 18 %), sei es in Form des Wassergehalts eines Oligoglucosesirups.

Das in der beschriebenen Weise gewonnene 1-phasige erste Reaktionsprodukt kann als solches der nachfolgenden zweiten Verfahrensstufe zugeführt werden, es kann aber auch wenigstens partiell abgekühlt und-/oder zwischengelagert und/oder durch Adsorptionsmittel farblich aufgehellt werden. Eine Verfahrensvereinfachung kann darin liegen, die Temperatur dieses ersten Reaktionsgemisches auf Werte unter 100 °C zu senken, um damit die Weiterführung dieses Produktstroms unter Normaldruck zu ermöglichen.

In der nachfolgenden zweiten Verfahrensstufe wird dieses erste Reaktionsprodukt mit den auf erhöhte Temperaturen vorgeheizten längerkettigen Alkoholen unter bestimmt ausgewählten Reaktionsbedingungen zusammengeführt. Für die Einstellung eines hohen Umsetzungsgrades und die Ausbildung von Alkylglucosidreaktionsprodukten gewünschter Farbe und gewünschten Aufbaus ist es wichtig, die Vereinigung des ersten Reaktionsproduktes mit den vorgeheizten längerkettigen Alkoholen unter solchen Reaktionsbedingungen von Druck, Temperatur und Zugabegeschwindigkeit vorzunehmen, daß der jeweils eingebrachte Wasseranteil praktisch unmittelbar in die Dampfphase übergeht. Die Erfindung will also die immerhin doch beträchtlichen Wassermengen aus der ersten Verfahrensstufe im zweiten Verfahrensabschnitt so rasch wie möglich aus dem Reaktionsgeschehen entfernen, um damit die gewünschte Umacetalisierung unter Verfahrensbedingungen sicherzustellen, die zu qualitativ hochwertigen Verfahrensendprodukten führen.

Von den längerkettigen Alkoholen mit 8 bis 22 C-Atomen sind die aliphatischen primären $C_{12}$- bis $C_{18}$-Alkohole besonders wichtig. Dabei handelt es sich vorzugsweise um gesättigte und insbesondere um geradkettige Alkohole, wie sie durch die Hydrierung von nativen Fettsäuren im technischen Maßstab erhalten werden können. Typische Vertreter der höheren aliphatischen Alkohole, die nach dem erfindungsgemäßen Verfahren verwendet werden können, sind z. B. die Verbindungen n-Dodecylalkohol, n-Tetradecylalkohol, n-Hexadecylalkohol, n-Octadecylalkohol, Eicosylalkohol, n-Octylalkohol, n-Decylalkohol, Undecylalkohol, Tridecylalkohol

bzw. ungesättigte Alkohole wie Oleylalkohol und Erucaalkohol. Da die Fettalkohole bevorzugt aus natürlichen Fettquellen stammen, kommen üblicherweise auch Gemische technischer Fettalkohole als Reaktionspartner in Betracht. Neben den eigentlichen Fettalkoholen sind auch verzweigtkettige primäre Alkohole, wie z. B. die sogenannten Oxoalkohole für die Umsetzung geeignet. Typische Oxoalkohole sind z.B. die Verbindungen $C_{12}/C_{13}$-Alkanol mit ca. 25 % hauptsächlich 2-Methylverzweigung (Dobanol 23), und der entsprechende $C_9$-$C_{11}$-Alkanol (Dobanol 91). Allerdings liegt ein Schwerpunkt des Verfahrens bei der Herstellung von Tensiden, die ausschließlich aus nachwachsenden Rohstoffen herstellbar sind.

In der bevorzugten Ausführungsform werden dazu die längerkettigen Alkohole wenigstens auf Verfahrenstemperatur der zweiten Reaktionsstufe vorgeheizt. Jetzt wird absatzweise oder kontinuierlich das hoch wasserhaltige erste Reaktionsprodukt so mit der heißen reaktiven Alkoholphase zusammengeführt, daß der jeweils eingebrachte Wasseranteil praktisch unmittelbar die Flüssigphase verläßt und in die Dampfphase übergeht. Dieser Verfahrensschritt wird durch die mitverwendeten Anteile der niederen monofunktionellen Alkohole und insbesondere durch das n-Butanol unterstützt. Das Wasser geht mit diesem Alkohol als Azeotrop über. n-Butanol bildet bekanntlich ein besonders wasserreiches heterogenes Azeotrop, das nach der Kondensation zur spontanen Abtrennung einer wäßrigen Phase führt, die im erforderlichen Ausmaß aus dem Verfahren abgezogen wird, während die sich im Kondensat bildende Butanolphase und das Restwasser in den Verfahrenskreislauf zurückgeführt werden können.

Zur optimalen Verwirklichung dieser Verfahrensbedingung kann es wünschenswert sein, das wasserhaltige erste Reaktionsprodukt unmittelbar in die vorgeheizte Hauptmenge des höheren Alkohols einzuspeisen und die spontan frei werdende Dampfphase mittels Kondensation aus dem Reaktionsgeschehen abzuziehen.

Eine wichtige Bedingung für die störungsfreie Durchführung des erfindungsgemäßen Verfahrens ist, daß auch in diesem zweiten Verfahrensschritt während des gesamten Reaktionsablaufs bei Verfahrenstemperatur im Flüssiganteil die Bedingung der 1-Phasigkeit gewährleistet ist. Diese Bedingung kann durch Steuerung insbesondere der Zugabegeschwindigkeit des wäßrigen ersten Reaktionsproduktes und die Temperaturkontrolle eingestellt und überwacht werden. Auch in dieser Verfahrensstufe kann die hinreichend intensive Vermischung des flüssigen Reaktionsgutes vorteilhaft sein.

Der zweiten Reaktionsstufe sind beträchtliche Energiemengen zur Verdampfung der Wasser/Butanol-Anteile aus dem ersten Reaktionsprodukt zuzuführen. Hier kommt der konkreten Ausgestaltung der Wärmezufuhr zur Herstellung hochwertiger Verfahrensendprodukte besondere Bedeutung zu. Dabei ist in einer bevorzugten Ausführungsform vorgesehen, bei dieser Zufuhr von Wärmeenergie zum zweiten Reaktionsgemisch eine sehr beschränkte Temperaturdifferenz $\Delta T$ (delta T) nicht zu überschreiten.

$\Delta T$ errechnet sich als der Differenzbetrag zwischen T (Heizmedium) - T (zweites Reaktionsgemisch). Der Absolutbetrag von $\Delta T$ soll in der bevorzugten Ausführungsform höchstens 30 °C betragen und bevorzugt nicht mehr als 20 °C ausmachen. In der Praxis werden besonders gute Ergebnisse erhalten, wenn man mit einem $\Delta T$ im Bereich von 10 bis 20 °C arbeitet.

Um diese wichtige Verfahrensbedingung trotz des vergleichsweise großen Anspruchs an Wärmezufuhr zur zweiten Reaktionsstufe bewirken zu können, wird insbesondere hier die benötigte Verdampfungsenergie über die Aufheizung eines im Kreislauf geführten Teilstromes dieses Reaktionsgemisches außerhalb des Hauptreaktors zugeführt. In diesem Kreislaufsystem ist außerhalb des Hauptreaktors ein so großer Wärmeaustauscher vorgesehen, daß die benötigten großen Mengen an Verdampfungsenergie praktisch spontan ersetzt bzw. zugeführt werden können, ohne die erfindungsgemäße wichtige Verfahrensbedingung des Maximums für $\Delta T$ zu überschreiten.

Die zweite Reaktionsstufe wird bevorzugt bei Temperaturen dicht unterhalb der ersten Reaktionsstufe, d. h. im Bereich von 100 bis 140 °C durchgeführt, wobei hier Temperaturen im Bereich von etwa 110 bis 120 °C bevorzugt sein können. Der Reaktionsverlauf in dieser Stufe der Umacetalisierung verlangt eine gewisse Verweildauer des Reaktionsgutes in dieser Verfahrensstufe. Dabei wird zunächst das wasserhaltige erste Reaktionsprodukt eingespeist und die praktisch sofortige Austreibung des Wasseranteils aus dem entstehenden zweiten Reaktionsgemisches sichergestellt. Es schließt sich eine Nachreaktionsphase der zunehmenden Umacetalisierung an, wobei im Reaktionsgemisch noch vorliegendes und/oder durch Umacetalisierung freigesetztes Niedrig-Alkanol insbesondere also n-Butanol - abgedampft wird. Dieser Verfahrensschritt kann durch stufenweises oder kontinuierliches Absenken des Drucks in dieser Reaktionsstufe gefördert werden. Bevorzugt wird dabei eine so weit führende Absenkung des Drucks bei dieser Umacetalisierung angestrebt, daß die Enddrucke dieser Verfahrensstufe unterhalb 100 mbar, bevorzugt unterhalb etwa 50 mbar und insbesondere im Bereich von etwa 10 mbar liegen. Das abdestillierte Butanol kann wiederum in den Verfahrenskreislauf zurückgegeben werden.

Die erfindungsgemäße Verfahrensführung sieht vor, daß praktisch während des gesamten Verfahrensabschnittes der Zugabe des ersten Reaktionsproduktes zum vorgeheizten längerkettigen Alkohol bzw. Alkoholgemisch die längerkettigen Alkohole im beträchtlichen Überschuß vorhanden sind. Hierdurch wird die Bildung der insbesondere gewünschten hohen Ausbeuten an Monoalkylglucosid stark gefördert. Aus diesem Grunde können im erfindungsgemäßen Verfahren die für die Umacetalisierung einzusetzenden Alkoholmengen im Vergleich mit vorbekannten Verfahren reduziert werden, ohne die Ausbildung der gewünschten hohen Anteile an Monoalkylglucosid negativ zu beeinflussen. Erfindungsgemäß ist es dementsprechend bevorzugt, im gesamten Ansatz des zweiten Reaktionsgemisches Molverhältnisse von eingesetzter Saccharidverbindung (bezogen auf Anhydroglucose) zum längerkettigen Alkohol im Bereich von 1 : 1,5 bis 7, vorzugsweise 1 : 2,5 bis 7, und insbesondere im Bereich von etwa 1 : 3 bis 5 zu verwenden. Hier liegt für den in der Regel nachfolgenden Verfahrensschritt der Abreicherung des zweiten Reaktionsproduktes an freiem längerkettigen Alkohol eine wichtige Verfahrensverbesserung.

Auch die Umacetalisierung der zweiten Verfahrensstufe wird sauer katalysiert durchgeführt. Es kann dabei zweckmäßig sein, den mit dem ersten Reaktionsprodukt übergetragenen Anteil an saurem Katalysator durch Zugabe zusätzlicher Katalysatormengen in die zweite Verfahrensstufe zu verstärken. Insbesondere sind auch hier wieder Sulfonsäuren, insbesondere aromatische Sulfonsäuren und ganz besonders die p-Toluolsulfonsäure geeignete katalytisch aktive Verbindungen. Als Alkoholeinsatzmaterialien für die zweite Reaktionsstufe sind längerkettige Alkohole bzw. Alkoholgemische natürlichen und/oder synthetischen Ursprungs, insbesondere primäre Alkanole, geeignet. Besondere Bedeutung kann den nachwachsenden Fettalkoholen zukommen, die durch Reduktion der Fettsäuren natürlichen Ursprungs erhalten werden können. Genau so sind aber entsprechende Alkohole bzw. Alkoholgemische synthetischen Ursprungs, wie zum Beispiel die Oxoalkohole, geeignet. Die Auswahl der jeweiligen Kettenlänge der eingesetzten Alkohole bzw. Alkoholgemische wird durch die jeweils geforderten tensidischen Eigenschaften der Alkylglucoside bestimmt. Für breite Anwendungsgebiete sind hier Kettenlängen von $C_{12-18}$ besonders geeignet.

Das aus der zweiten Reaktionsstufe anfallende Reaktionsprodukt wird in an sich bekannter Weise weiter gereinigt und aufgearbeitet, wie es im einzelnen in der eingangs geschilderten Literatur und dabei auch insbesondere in der genannten Anmeldung P 37 23 826.4 geschildert ist.

In Betracht kommt hier dementsprechend zunächst die Neutralisation des sauer katalysierten Reaktionsprodukts. Geeignet hierfür sind organische oder anorganische basische Alkali-, Erdalkalioder Aluminium- bzw. Alkali/Aluminiumverbindungen. Zweckmäßigerweise werden dabei pH-Werte von wenigstens 8, vorzugsweise von etwa 9 eingestellt. Zur Neutralisation des Katalysators sind insbesondere Magnesiumverbindungen, beispielsweise Organomagnesiumverbindungen wie Magnesiumalkoholate oder anorganische Magnesiumverbindungen wie Magnesiumoxid bzw. Magnesiumhydroxid geeignet. Ein brauchbares Neutralisationsmittel ist aber auch Zeolith NaA, insbesondere in Abmischung mit Calciumhydroxid. Die Einstellung alkalischer Werte in dieser Neutralisationsstufe stellt die Farbstabilität des Tensids im Alkalischen bei seiner späteren Verarbeitung sicher.

Die Abdestillation des Fettalkoholüberschusses kann in bekannten produktschonenden Vakuumdestillationsvorrichtungen erfolgen. Geeignet ist hier insbesondere der Einsatz von Dünnschichtund/oder Fallfilmverdampfern.

Das Reaktionsendprodukt bildet im erkalteten Zustand eine wachsartige Masse, die in an sich bekannter Weise zur besseren Handhabbarkeit in wäßrigen Pasten mit Wirkstoffgehalten oberhalb 50 % überführt werden. In dieser Form kann insbesondere - soweit erforderlich durch eine einfache Bleiche mit Wasserstoffperoxid oder einer organischen Persäure bzw. entsprechenden Persäuresalzen eine zusätzliche Farbkorrektur und Farbaufhellung erfolgen.

Die im erfindungsgemäßen Verfahren anfallenden Stoffgemische entsprechen ihrer Zusammensetzung der Offenbarung aus der genannten deutschen Patentanmeldung P 37 23 826.4, auf deren Angaben zum Zwecke der vollständigen Erfindungsoffenbarung einfachheitshalber verwiesen wird.

**Beispiele**

Beispiel 1

Kartoffelstärke wird im erfindungsgemäßen Mehrstufenverfahren zu einem oberflächenaktive Alkylglucosidverbindungen enthaltenden Reaktionsprodukt verarbeitet. Dabei kommen die folgenden Einsatzstoffe in den nachfolgend angegebenen Mengen zur Verwendung:

250 kg      Stärkepulver (Wassergehalt 18 %), entsprechend 205 kg Stärke (wasserfrei) + 45 kg Wasser
795 kg      n-Butanol
1480 kg      $C_{12/14}$-Fettalkohol (native Basis)
135 kg      Zusatzwasser (Gesamtwasser aus Stärkeeinsatz + Zusatz Wasser 180 kg)
7,6 kg      p-Toluolsulfonsäure x 1 $H_2O$, davon Einsatz im ersten Reaktor 5,6 kg, die restlichen 2,0 kg werden

EP 0 357 969 B1

dem zweiten Reaktor beigegeben

4,2 kg      Magnesiumoxid zur Neutralisation

Die Mengenverhältnisse der Einsatzmaterialien entsprechen den folgenden Molverhältnissen - jeweils bezogen auf 1 Mol wasserfreie Kartoffelstärke (berechnet als Anhydroglucose):

8 Mol Wasser, 8,5 Mol Butanol, 6 Mol $C_{12/14}$-Fettalkohol, $3,16 \times 10^{-2}$ Mol p-Toluolsulfonsäure und $8,22 \times 10^{-2}$ Mol Magnesiumoxid

Der Reaktionsablauf wird im einzelnen wie folgt geführt:

Butanol und Wasser werden in einem ersten Reaktor vorgelegt. Dieser erste Reaktor ist als Rührreaktor mit einem Volumen von 2,5 m³ ausgelegt, mit einem 3-stufigen Rührwerk mit Austragsflügel versehen und enthaltend zusätzlich einen Supraton-Inline-Mischer mit einer Leistung von 2 m³/h. Dieser erste Reaktionsbehälter weist darüber hinaus einen externen Flüssigkeitskreislauf mit einem Wärmeaustauscher auf.

Das im Reaktor vorgelegte Butanol/Wasser-Gemisch wird mit Hilfe des Inline-Mischers im Kreislauf gepumpt. Die pulverförmige Stärke und der erste Anteil des sauren Katalysators (5,6 kg p-Toluolsulfonsäure) werden zu dem Butanol/Wasser-Gemisch gegeben. Das Reaktionsgemisch wird auf die Reaktionstemperatur von 140 bis 145 °C unter Eigendruck aufgeheizt, dabei wird die beheizbare Kreislaufleitung zur Wärmezufuhr eingesetzt. Der sich im geschlossenen System einstellende Eigendruck liegt bei 4,3 bis 4,7 bar. Nach einer Reaktionszeit von 0,33 h bei einer Reaktionstemperatur von 140 bis 145 °C wird das Reaktionsgemisch auf 70 bis 80 °C abgekühlt.

In einem zweiten Reaktionsbehälter werden der $C_{12/14}$-Fettalkohol und zusätzlich 2 kg p-Toluolsulfonsäure vorgelegt. Der zweite Reaktionsbehälter ist als Rührreaktor mit einem Volumen von 3,2 m³ ausgelegt und mit einer Rektifizierkolonne versehen. Zusätzlich ist ein externer Flüssigkeitskreislauf vorgesehen, der eine Kreiselpumpe einer Leistung von 20 m³/h und einen Rohrbündelwärmeaustauscher mit einer Austauscherfläche von 20 m² aufweist.

Das Fettalkohol/Katalysator-Gemisch wird durch den externen Kreislauf gepumpt und auf eine Reaktionstemperatur von 115 bis 118 °C aufgeheizt. Am Reaktor wird ein Druck von 700 mbar angelegt. Sobald die vorgegebene Reaktionstemperatur und der Reaktionsdruck eingestellt sind, wird innerhalb von 2 Stunden das Reaktionsgemisch aus dem ersten Reaktor kontinuierlich in den zweiten Reaktor dosiert.

Butanol und Wasser werden kontinuierlich und möglichst schnell aus dem Reaktionsgemisch entfernt. Die dafür erforderliche Verdampfungsenergie wird über den Wärmeaustauscher des externen Flüssigkeitskreislaufs bei einem $\Delta$T-Wert nicht über 20 °C zugeführt.

Das aus der abgetrennten Dampfphase gewonnene Kondensat entmischt sich in eine schwere wasserreiche Phase (92,3 % Wasser, 7,7 % Butanol) und eine überstehende butanolreiche Phase. 45 kg Wasser, die durch den natürlichen Wassergehalt der Stärke in das Reaktionsgemisch gelangt sind, werden als schwere Phase aus dem System abgezogen. Das restliche Wasser und das Butanol werden in einer Vorlage gesammelt und im nachfolgenden Ansatz dem Verfahren wieder zugeführt.

Nach beendeter Dosierung des Reaktionsgemisches aus dem ersten Reaktor in den zweiten Reaktor wird jetzt noch vorliegendes bzw. durch Umacetalisierung freigesetztes Butanol möglichst vollständig aus dem zweiten Reaktor entfernt. Hierzu wird der Druck im Reaktor schrittweise auf einen Enddruck von etwa 10 mbar abgesenkt. Der benötigte Zeitraum beträgt etwa 1 Stunde.

Nach beendeter Umacetalisierung wird das Reaktionsgemisch auf 90 °C abgekühlt und mit dem Magnesiumoxid neutralisiert. Nachfolgend wird in an sich bekannter Weise der Überschuß des $C_{12/14}$-Fettalkohols auf destillativem Wege abgereichert, das so gewonnene Produkt zu einer wäßrigen Paste mit einem Wirkstoffgehalt von ca. 60 % aufgearbeitet und durch Zusatz von Wasserstoffperoxid gebleicht.

In nachfolgenden Reaktionsansätzen wird unter Beibehaltung der beschriebenen Molverhältnisse anstelle der Kartoffelstärke zunächst gebleichte Maisstärke und dann in einem weiteren Ansatz ungebleichte Maisstärke - jeweiliger Wassergehalt 11 bis 13 % - verarbeitet. Die Aufarbeitung auch dieser natürlichen Stärkematerialien erfolgt problemlos und führt zu überwiegend Monoalkylglucoside enthaltenden Reaktionsprodukten vergleichbarer Qualität.

Beispiel 1a

Es wurde wie oben beschrieben gearbeitet, wobei in der ersten Reaktionsstufe der Eigendruck im Bereich 5,0 bis 5,5 bar betrug.

Beispiel 2

Das Verfahrensprinzip des Beispiels 1 wird wiederholt, es kommt jetzt jedoch ein Glucosesirup als Polysaccharidmaterial zum Einsatz, der einen Wassergehalt von 20 Gew.-% und einen Gehalt an Glucose von ebenfalls ca. 20 % aufweist und zum Rest aus Oligo- bzw. Polysaccharidkomponenten besteht. Die Einsatzrohstoffe sind mengenmäßig aufgelistet wie folgt:

256,3 kg        Glucosesirup (20 % $H_2O$, ca. 20 % Glucose)

795,0 kg        n-Butanol

1397,0 kg       $C_{12/14}$-Fettalkohol

128,7 kg        Zusatz an vollentsalztem Wasser (zusätzlich Wasser ex-Glucosesirup 51,3 kg)

7,6 kg          p-Toluolsulfonsäure x 1 $H_2O$, davon 5,6 kg im ersten Reaktor, 2,0 kg zusätzlich im zweiten Reaktor

4,2 kg          Magnesiumoxid

Es wird analog den Verfahrensangaben des Beispiels 1 gearbeitet. Dabei sind die Reaktionsbedingungen wie folgt gewählt:

Erster Reaktor 145 °C, 4,8 bis 5,3 bar bei einer Reaktionszeit von 0,4 bis 0,6 Stunden

Zweiter Reaktor 113 bis 118 °C, abnehmender Druck von 700 bis 10 mbar, Reaktionszeit 3 Stunden

Das wie in Beispiel 1 aufgearbeitete Reaktionsprodukt entspricht in Aussehen und Beschaffenheit dem aus Kartoffelstärke gewonnenen Alkylglucosid-Reaktionsprodukt.

Beispiel 3

Dieses Beispiel beschreibt einen Laboransatz des erfindungsgemäßen Verfahrens. Die molaren Umsetzungsverhältnisse Stärke : Butanol : Wasser : Fettalkohol betrugen 1 : 8 : 6 : 4, wobei die Stärkemenge auf 1 Mol Anhydroglucose, MG = 162, bezogen wurde.

In der ersten Stufe wurde die 15 % Wasser enthaltende Kartoffelstärke (93,5 g bzw. 0,5 Mol) mit 296,5 g (4 Mol) n-Butanol, 39,7 g Wasser (mit dem Wasser aus der Stärke insgesamt 3 Mol) und 1,6 g p-Toluolsulfonsäure gemischt und im Autoklaven eine halbe Stunde bei 145 °C gerührt (190 U/min). Dabei stieg der Druck auf 3,7 bar. Nach Abkühlen auf ca. 80 °C wurde das gelbbräunliche klare Reaktionsgemisch abgelassen.

In der zweiten Verfahrensstufe wurde der Fettalkohol (388 g bzw. 2 Mol Dodecanol/Tetradecanol im Gewichtsverhältnis 75 : 25) zusammen mit 1,6 g p-Toluolsulfonsäure in einem 1-Liter-Dreihalskolben mit Rührer, Tropftrichter und Destillationskolonne mit Destillationsbrücke vorgelegt und unter Rühren auf 112 °C erhitzt. Nachdem ein Vakuum von 15 mmHg angelegt worden war, wurde das ca. 80 °C warme Produkt der Stufe 1 in 1 1/2 Stunden hinzugegeben und gleichzeitig ein Butanol/Wasser-Gemisch abdestilliert. Danach wurde das Vakuum auf 3 mmHg eingestellt und die Butanol-Restmenge während ca. 1 Stunde abgezogen. Das Reaktionsgemisch wurde dann auf ca. 90 °C abgekühlt und mit 2,0 g Magnesiumoxid versetzt. Anschließend wurde der überschüssige Fettalkohol bei 0,05 - 0,01 mmHg unter Rühren abdestilliert. Die maximale Sumpftemperatur betrug 165 °C. Es wurden 334 g Fettalkohol erhalten. Der Destillationsrückstand betrug 136 g. Das so erhaltene Produkt wurde bei ca. 90 °C mit Wasser in eine etwa 50%ige Paste übergeführt und durch Zugabe von 30%iger Wasserstoffperoxidlösung (1 % $H_2O_2$, bezogen auf die Produktmenge) gebleicht. Es wurde eine gelbliche Masse erhalten, die im alkalischen Milieu farbstabil war (Farbzahlen nach Klett, 5%ige Lösung in Wasser/Isopropylalkohol 1 : 1, 1cm-Küvette, Blaufilter: 30 vor und 40 nach Alkalifarbtest).

**Patentansprüche**

1.      Verfahren zur Herstellung von oberflächenaktive Alkylglucosidverbindungen enthaltenden Reaktionsprodukten mit überwiegendem Gehalt an Alkyl-Monoglucosid aus Oligo- und/oder Polysacchariden, insbesondere Stärke oder partiellen Stärkeabbauprodukten, und längerkettigen monofunktionellen Alkoholen unter Einschluß einer intermediären Bildung von Niedrig-Alkyl-Glucosiden und deren Umacetalisierung mit den längerkettigen monofunktionellen Alkoholen, dadurch gekennzeichnet, daß man

a) das Saccharid-Einsatzmaterial mit solchen Mengen einer bei Verfahrenstemperatur 1-phasigen Wasser/Niedrig-Alkanol-Mischung bei Temperaturen oberhalb 100 °C und erhöhten Drucken sauer katalysiert umsetzt, daß während des ganzen Reaktionsablaufs die Rühr- und Fließfähigkeit des Reaktionsgemisches erhalten bleibt und dabei die Umsetzung so weit führt, daß ein flüssiges, praktisch klares 1-phasiges erstes Reaktionsprodukt anfällt, woraufhin man

b) dieses erste Reaktionsprodukt, gegebenenfalls nach Vorbehandlung mit einem Adsorptionsmittel, mit den auf erhöhte Temperaturen vorgeheizten längerkettigen Alkoholen ebenfalls unter Ausbildung eines während des gesamten Reaktionsablaufs bei Verfahrenstemperatur im Flüssiganteil 1-phasigen zweiten Reaktionsgemisches unter solchen Reaktionsbedingungen von Druck, Temperatur und Zugabegeschwindigkeit zusammenführt, daß der jeweils eingebrachte Wasseranteil praktisch unmittelbar in die Dampfphase übergeht und dort zusammen mit begleitendem Niedrig-Alkanol kondensiert und damit dem Reaktionsgeschehen entzogen wird, woraufhin

c) unter Aufrechterhaltung der 1-Phasigkeit des Flüssiganteils darin noch vorliegendes und/oder durch Umacetalisierung freigesetztes Niedrig-Alkanol abgedampft und das so gebildete zweite Reaktionsprodukt gewünschtenfalls in an sich bekannter Weise - insbesondere durch Neutralisation, Entfernung überschüssigen längerkettigen Alkohols und/oder Bleiche - weiterführend gereinigt wird.

2. Verfahren nach Anspruch 1, dadurch gekennzeichnet, daß man bei der Zufuhr von Wärmeenergie zum zweiten Reaktionsgemisch mit einer Temperaturdifferenz $\Delta T = T_{Heizmedium} - T_{2.Reaktionsgemisch}$ von höchstens 30 °C, bevorzugt nicht mehr als 20 °C arbeitet.

3. Verfahren nach einem der Ansprüche 1 oder 2, dadurch gekennzeichnet, daß man den wenigstens überwiegenden Anteil der im zweiten Reaktionsgemisch benötigten Verdampfungsenergie über die Aufheizung eines im Kreislauf geführten Teilstromes dieses Reaktionsgemisches außerhalb des Hauptreaktors zuführt.

4. Verfahren nach einem der Ansprüche 1 bis 3, dadurch gekennzeichnet, daß auch die Beheizung des ersten Reaktionsgemisches an einem im Kreislauf geführten Teilstrom vorgenommen wird.

5. Verfahren nach einem der Ansprüche 1 bis 4, dadurch gekennzeichnet, daß man mit $C_3$-$C_5$ Monoalkoholen, insbesondere mit n-Butanol als Niedrig-Alkanol arbeitet.

6. Verfahren nach einem der Ansprüche 1 bis 5, dadurch gekennzeichnet, daß man pulverförmige native Stärke oder Polyglucose- oder Glucose-Sirup als Einsatzmaterial verwendet.

7. Verfahren nach einem der Ansprüche 1 bis 6, dadurch gekennzeichnet, daß man den Saccharidaufschluß der ersten Reaktionsstufe bei Temperaturen oberhalb 125 °C, vorzugsweise im Bereich von etwa 135 bis 170 °C und bevorzugt unter dem sich im geschlossenen System ausbildenden Eigendruck von insbesondere etwa 4 bis 10 bar durchführt.

8. Verfahren nach einem der Ansprüche 1 bis 7, dadurch gekennzeichnet, daß die zweite Reaktionsstufe bei Temperaturen dicht unterhalb denen der ersten Reaktionsstufe durchgeführt wird, wobei Temperaturen im Bereich von 110 bis 120 °C bevorzugt sind und wobei insbesondere in dieser Reaktionsstufe unter vermindertem Druck gearbeitet wird.

9. Verfahren nach einem der Ansprüche 1 bis 8, dadurch gekennzeichnet, daß in der Reaktionsstufe der Umacetalisierung mit zunehmendem Vakuum - bevorzugter Enddruck bei etwa 10 mbar - gearbeitet wird.

10. Verfahren nach einem der Ansprüche 1 bis 9, dadurch gekennzeichnet, daß die in die zweite Reaktionsstufe übergegangene Katalysatormenge durch Zusatz weiterer Anteile sauren Katalysators - in beiden Stufen bevorzugt Sulfonsäure - angehoben wird.

11. Verfahren nach einem der Ansprüche 1 bis 10, dadurch gekennzeichnet, daß zum Ansetzen des ersten Reaktionsgemisches Wasser und Butanol in etwa gleichen Molmengen zum Einsatz kommen.

12. Verfahren nach einem der Ansprüche 1 bis 11, dadurch gekennzeichnet, daß im ersten Reaktionsgemisch Molverhältnisse von Saccharidverbindung, insbesondere Stärke : Wasser im Bereich von etwa 1 : 5 bis 12 verwendet werden.

13. Verfahren nach einem der Ansprüche 1 bis 12, dadurch gekennzeichnet, daß im Gesamtansatz des zweiten Reaktionsgemisches Molverhältnisse von Ausgangsstärke : längerkettigen Alkoholen im Bereich von 1 : 1,5 bis 7, vorzugsweise von 1 : 2,5 bis 7 und insbesondere 1 : 3 bis 5 verwendet werden.

14. Verfahren nach Ansprüchen 1 bis 13, dadurch gekennzeichnet, daß zur Neutralisation des sauren Katalysators dem Reaktionsgemisch nach Abschluß der Umsetzung organische und/oder anorganische basische Alkali-, Erdalkali- oder Aluminiumverbindungen zugegeben werden, wobei die Einstellung von pH-Werten von wenigstens 8 im Reaktionsprodukt bevorzugt ist.

15. Verfahren nach Ansprüchen 1 bis 14, dadurch gekennzeichnet, daß das Reaktionsrohprodukt einer oxidativen Bleiche mit insbesondere Wasserstoffperoxid und/oder Persäuren bzw. Persäuresalzen unterworfen wird.

16. Verfahren nach Ansprüchen 1 bis 15, dadurch gekennzeichnet, daß als längerkettige monofunktionelle Alkohole entsprechende Verbindungen natürlichen und/oder synthetischen Ursprungs des überwiegenden Kettenlängenbereichs von $C_{12-18}$ eingesetzt werden.

**Claims**

1. Process for preparing reaction products containing surface active alkylglucoside compounds, with a predominant content of alkylmonoglucoside from oligo- and/or polysaccharides, particularly starch or partial starch degradation products, and longer chained monofunctional alcohols, with the inclusion of the intermediate formation of low alkylglucosides and the transacetalisation thereof with the longer chained monofunctional alcohols, characterised in that

a) the saccharide starting material is reacted, with acidic catalysis, with such quantities of a water/low alkanol mixture which is in a single phase at the operating temperature, at temperatures above 100°C and elevated pressures, so that throughout the entire reaction the stirability and flowability of the reaction mixture are maintained and the reaction is continued until a liquid, virtually clear single-phase first reaction product is obtained, after which

b) this first reaction product, optionally after preliminary treatment with an adsorbant, is combined with the longer-chained alcohols preheated to elevated temperatures, again with the formation of a second reaction mixture which is single-phase at the operating temperature in the liquid component throughout the entire reaction, under such reaction conditions of pressure, temperature and rate of addition that the water content introduced is converted into the vapour phase virtually immediately and there is condensed together with the accompanying low alkanol and hence removed from the events of the reaction, after which

c) whilst the single phase nature of the liquid component is maintained, any low alkanol still present therein and/or liberated by transacetalisation is evaporated off and the second reaction product thus formed is, if desired, further purified in a manner known per se, more particularly by neutralisation, elimination of excess longer-chained alcohol and/or bleach.

2. Process according to claim 1, characterised in that when the heat energy is supplied to the second reaction mixture a temperature difference

$$\Delta T = T_{heating\ medium} - T_{second\ reaction\ mixture}$$

of not more than 30°C, preferably not more than 20°C, is used.

3. Process according to one of claims 1 and 2, characterised in that at least the majority of the vaporising energy required in the second reaction mixture is supplied by heating a partial current of this reaction mixture circulated outside the main reactor.

4. Process according to one of claims 1 to 3, characterised in that the heating of the first reaction mixture is also carried out on a circulating partial current.

5. Process according to one of claims 1 to 4, characterised in that $C_{3-5}$ monoalcohols, particularly n-butanol, is used as the low alkanol.

6. Process according to one of claims 1 to 5, characterised in that powdered native starch or polyglucose or glucose syrup is used as a starting material.

7. Process according to one of claims 1 to 6, characterised in that the saccharide digestion of the first reaction step is carried out at temperatures above 125°C, preferably in the range from about 135 to 170°C and preferably under the inherent pressure of, in particular, about 4 to 10 bar established in the closed system.

8. Process according to one of claims 1 to 7, characterised in that the second reaction step is carried out at temperatures just below those of the first reaction step, temperatures in the range from 110 to 120°C being preferred and this reaction step in particular being carried out under reduced pressure.

9. Process according to one of claims 1 to 8, characterised in that, in the reaction step of transacetalisation, the operation is carried out with an increasing vacuum, preferably with a final pressure of about 10 mbar.

10. Process according to one of claims 1 to 9, characterised in that the quantity of catalyst transferred into the second reaction step is increased by the addition of further amounts of acid catalyst - in both steps preferably sulphonic acid.

11. Process according to one of claims 1 to 10, characterised in that water and butanol are used in substantially the same molar amounts to make up the first reaction mixture.

12. Process according to one of claims 1 to 11, characterised in that in the first reaction mixture molar ratios of saccharide compound, particularly starch, to water in the range from about 1:5 to 12 are used.

13. Process according to one of claims 1 to 12, characterised in that in the total composition of the second reaction mixture, molar ratios of starting starch to longer chained alcohols ranging from 1:1.5 to 7, preferably from 1:2.5 to 7 and particularly 1:3 to 5, are used.

14. Process according to claims 1 to 13, characterised in that in order to neutralise the acid catalyst, organic and/or inorganic basic alkaline, alkaline earth metal or aluminium compounds are added to the reaction mixture after the end of the reaction, whilst it is preferable for pH values of at least 8 to be established in the reaction product.

15. Process according to claims 1 to 14, characterised in that the crude reaction product is subjected to oxidative bleaching with, in particular, hydrogen peroxide and/or per acids or salts of per acids.

16. Process according to claims 1 to 15, characterised in that corresponding compounds of natural and/or synthetic origin with chain lengths predominantly in the range from $C_{12-18}$ are used as the longer chained monofunctional alcohols.

**Revendications**

1°) Procédé de production de produits de réaction contenant des composés alkylglucosides tensioactifs avec une teneur majoritaire en alkylmonoglucoside d'oligo et/ou polysaccharides, notamment de l'amidon ou des produits de dégradation partielle de l'amidon, et d'alcools monofonctionnels à longue chaîne en incluant une formation intermédiaire d'(alkyle inférieur)-glucosides et leur transacétilasation avec les alcools monofonctionnels à longue chaîne, procédé caractérisé en ce que :
a) on fait réagir le matériau de base saccharide avec des quantités d'un mélange eau/alcanol inférieur monophasique à des températures supérieures à 100°C et sous forte pression en présence de catalyseur acide, de sorte que pendant tout le déroulement de la réaction, on conserve l'aptitude à l'écoulement et à l'agitation du milieu réactionnel, et on poursuit la réaction jusqu'à ce qu'on obtienne un premier produit de réaction monophasique, liquide pratiquement clair, et ensuite,
b) on fait réagir ce premier produit de réaction, le cas échéant après un prétraitement de décoloration avec un adsorbant avec des alcools à longue chaîne portés également à haute température, en formant un deuxième mélange réactionnel monophasique dans la partie liquide pendant le déroulement global de la réaction à la température du procédé, dans des conditions réactionnelles de pression, température et vitesse d'addition, telles que toute proportion d'eau incluse passe pratiquement directement dans la phase gazeuse et elle s'y condense avec l'alcanol inférieur et est ainsi soustraite au mélange réactionnel, et ensuite
c) en maintenant l'unicité de phase de la partie liquide l'alcanol inférieur encore existant et/ou libéré par transacétalisation est vaporisé, et le deuxième produit de réaction formé est purifié à la suite si on le souhaite, de manière connue en soi - notamment par neutralisation, élimination de l'alcool à longue chaîne en excès et/ou blanchiment.
2°) Procédé selon la revendication 1, caractérisé en ce que lors de l'apport d'énergie calorifique au deuxième mélange réactionnel, on travaille avec une différence de température $\Delta T = T_{milieu\ de\ chauffage} - T_{2ème\ mélange\ réactionnel}$ d'au plus 30°C, et qui ne dépasse pas 20°C de préférence.
3°) Procédé selon l'une des revendications 1 ou 2, caractérisé en ce qu'on introduit au moins la proportion essentielle d'énergie d'évaporation nécessaire dans le deuxième mélange réactionnel par chauffage d'un courant partiel de ce mélange réactionnel, à l'extérieur du réacteur principal qu'on réintroduit dans le circuit.
4°) Procédé selon l'une des revendications 1 à 3, caractérisé en ce qu'on réalise le chauffage du premier mélange réactionnel par un courant partiel qu'on réintroduit dans le circuit.
5°) Procédé selon l'une des revendications 1 à 4, caractérisé en ce qu'on travaille avec des monoalcools $C_3$-$C_5$, notamment avec le n-butanol comme alcanol inférieur.

6°) Procédé selon l'une des revendications 1 à 5, caractérisé en ce qu'on utilise comme matériau de base de l'amidon naturel en poudre ou du sirop de polyglucose ou de glucose.

7°) Procédé selon l'une des revendications 1 à 6, caractérisé en ce qu'on réalise la dissolution de saccharide de la première étape réactionnelle à des températures supérieures à 125°C, de préférence dans l'intervalle d'environ 135 à 170°C et de préférence sous la pression qui se développe dans le système clos, comprise entre environ 4 et 10 bars.

8°) Procédé selon l'une des revendications 1 à 7, caractérisé en ce qu'on réalise la deuxième étape de procédé à des températures juste en-dessous de celles de la première étape de réaction, en préférant des températures comprises entre 110 et 120°C et en travaillant sous pression réduite dans cette étape de réaction.

9°) Procédé selon l'une des revendications 1 à 8, caractérisé en ce que dans cette étape de réaction, on réalise la transacétalisation sous vide croissant - avec une pression finale préférée d'environ 10 mbars -.

10°) Procédé selon l'une des revendications 1 à 9, caractérisé en ce que lors de la deuxième étape de réaction, on ajoute à la quantité de catalyseur provenant de la première étape une autre quantité de catalyseur acide - qui est de préférence dans les deux étapes de l'acide sulfonique.

11°) Procédé selon l'une des revendications 1 à 10, caractérisé en ce que pour réaliser le premier mélange réactionnel, on utilise de l'eau et du butanol en quantités molaires à peu près égales.

12°) Procédé selon l'une des revendications 1 à 11, caractérisé en ce que dans le premier mélange réactionnel, on utilise des proportions molaires de composé saccharide, notamment amidon : eau dans l'intervalle d'environ 1 : 5 à 12.

13°) Procédé selon l'une des revendications 1 à 12, caractérisé en ce que dans le lot total du deuxième mélange réactionnel, on utilise des proportions molaires d'amidon de départ : alcools à longue chaîne dans l'intervalle de 1 : 1,5 à 7, de préférence de 1 : 2,5 à 7 et notamment 1 : 3 à 5.

14°) Procédé selon les revendications 1 à 13, caractérisé en ce que pour neutraliser le catalyseur acide, on ajoute au mélange réactionnel à la fin de la réaction des composés basiques organiques et/ou minéraux alcalins, alcalino-terreux ou d'aluminium, en préférant ajuster le pH à au moins 8 dans le produit de la réaction.

15°) Procédé selon les revendications 1 à 14, caractérisé en ce qu'on soumet le produit de réaction à un blanchiment oxydant avec notamment du peroxyde d'hydrogène et/ou des peracides ou des sels de peracides.

16°) Procédé selon les revendications 1 à 15, caractérisé en ce qu'on utilise comme alcools monofonctionnels à longue chaîne des composés correspondants d'origine naturelle et/ou synthétique surtout dans le domaine de longueur de chaîne de $C_{12-18}$.